# EUROPEAN PATENT SPECIFICATION

(11) **EP 3 033 068 B1**
(45) Date of publication and mention of the grant of the patent: **21.11.2018**
(21) Application number: 14752306.2
(22) Date of filing: 13.08.2014
(51) Int. Cl.: A61K 8/24, A61K 8/43, A61Q 5/08, A61Q 5/10, A61K 8/19, A61K 8/41, A61K 8/22

(54) **AQUEOUS COMPOSITION FOR TREATING HAIR**
WÄSSRIGE ZUSAMMENSETZUNG ZUR HAARBEHANDLUNG
COMPOSITION AQUEUSE POUR LE TRAITEMENT DES CHEVEUX

(30) Priority: 15.08.2013 EP 13180531
(43) Date of publication of application: 22.06.2016
(73) Proprietor: Kao Corporation, Chuo-Ku Tokyo 103-8210 (JP)
(72) Inventor: SCHMELZ, Sandra, 97828 Marktheidenfeld (DE); FEIER-IOVA, Ovidiu, 64287 Darmstadt (DE); LEUWER, Somayeh Samy, 60327 Frankfurt (DE)
(74) Representative: Hoffmann Eitle
(86) International application number: PCT/EP2014/067323
(87) International publication number: WO 2015/022360

(56) References cited:
- EP-A1- 0 890 355
- EP-A2- 1 106 166
- EP-A2- 1 862 198
- DE-U1- 29 722 990
- GB-A- 1 384 768
- JP-A- 2002 068 976
- US-A- 4 226 852
- US-A1- 2009 142 288
- DATABASE CA [Online] CHEMICAL ABSTRACTS SERVICE, COLUMBUS, OHIO, US; 11 October 2012 (2012-10-11), "Oxidative hair dye or bleaching agent composition containing guanidinium salt and aromatic alcohol", XP002732385, Database accession no. 157:585970
- DATABASE GNPD [Online] MINTEL; April 2000 (2000-04), "Living Colors", XP002721679, Database accession no. 23585

## Description

### Field of the Invention

The present invention relates to an aqueous composition for treating hair, especially for lightening hair.

### Background of the Invention

Hair lighteners and bleaching compositions have been known for many decades. They usually comprise an oxidizing agent in an aqueous alkaline medium. They are very effective in bleaching natural hair color but have a relatively low level of lightening power on colored hair, especially oxidative colored hair. Therefore, lightening and/or bleaching artificially colored hair, especially oxidative colored hair remains a desire to be met.

Ineffective lightening of artificially colored hair causes problems when such hair is to be re-colored. The major problem is uneven coloration between the root part, which has the natural color and may be lightened effectively, and the length towards the hair ends, which has the artificial color form the previous coloration. The problem is aggravated when the color nature is desired to be changed.

In order to increase the lightening power of the composition alkaline carbonate and/or bicarbonate salts have been widely used. Indeed alkaline carbonate and/or bicarbonate salts increase the lightening effect, but they fail to lighten the color of artificially colored hair effectively and homogenously as it is the case for natural hair. Therefore, problems associated with coloring hair comprising artificially colored parts remain unchanged.

For example, EP 890 355 A1 discloses hair dyeing compositions comprising guanidium salt such as guanidium carbonate in an ammonia comprising composition. The document does not disclose anything on combining the guanidium salt with another alkaline salt.

EP 642 783 discloses oxidative hair dyeing compositions comprising ammonium salts. Among the suitable salts ammonium carbonate and ammonium bicarbonate are mentioned. However, the document fails to disclose any ammonia free composition and also has no teaching of compositions comprising two alkaline salts.

US 2009/142288 is directed to alkaline hair straightening compositions having a pH of 12 to 14 and including at least one hydroxide ion generator, a saccharide and a diacid. Sodium hydroxide may be used as the hydroxide ion generator, and guanidine carbonate may be optionally added.

JP 2002-068976 relates to an alkaline composition for cosmetic application, comprising guanidine hydroxide and guanidine carbonate, which may, e.g., be used as hair bleaching agent or hair dye. For this purpose, ammonium phosphate buffered hydrogen peroxide solution may be added as oxidizing agent.

GB 1384768 is directed to hair bleaching compositions including a guanidine compound in combination with an oxidizing agent. The pH may be adjusted to the alkaline range with a base such as monoethanolamine.

DE 297 22 990 U1 relates to an agent for lightening hair comprising a water soluble guanidine salt, which is provided in powder form, to be mixed with hydrogen peroxide solution before application. The hydrogen peroxide may be buffered, e.g., with phosphoric acid/sodium phosphate.

JP 2012-224621 is directed to an oxidative hair dye or bleaching agent composition, which is obtained by mixing a first agent containing an alkaline agent with a second agent containing hydrogen peroxide, wherein the mixture has a pH of 8.0-11.5 contains more than 0.5 mass% of a guanidium salt and more than 2.0 mass% of an aromatic alcohol which is liquid at ordinary temperature.

### Summary of the Invention

The present invention relates to an aqueous composition for hair, characterized in that it comprises at least one alkalizing agent selected from ammonia and compounds according to the general formula

R₁R₂R₃N

wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and is preferably a mono or polyhydroxyalkyl; at least one guanidine salt, which is selected from guanidine carbonate or guanidine phosphate; at least one alkaline salt selected from the salts of carbonate, bicarbonate, phosphate and hydrogenphosphates with sodium, potassium or ammonium; and at least one oxidizing agent; wherein the at least one guanidine salt and at least one alkaline salt are comprised in the composition at a total concentration of 3% or more, preferably 5 to 40% by weight, calculated to total composition: the weight ratio of the guanidine salt to the alkaline salt being 10:1 to 1:5; and wherein the composition is preferably free of persalts.

Furthermore, the present invention relates to a kit for hair, comprising two or more aqueous compositions wherein one of the compositions is an aqueous composition comprising at least one alkalizing agent selected from ammonia and compounds according to the general formula

R₁R₂R₃N

wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and is preferably a mono or polyhydroxyalkyl; at least one guanidine salt, which is selected from guanidine carbonate or guanidine phosphate; at least one alkaline salt selected from the salts of carbonate, bicarbonate, phosphate and hydrogenphosphates with sodium, potassium or ammonium; wherein at least one guanidine salt and at least one alkaline salt are comprised in the composition at a total concentration of 3% or more, preferably 5 to 40% by weight, calculated to total composition, the weight ratio of the guanidine salt to the alkaline salt being 10:1 to 1:5: and wherein the composition is preferably free of persalts; and the other composition is an aqueous composition comprising at least one oxidizing agent.

Besides, the present invention also relates to the use of the composition and the kit for treating, specifically bleaching, lightening or dyeing the hair.

### Detailed Description of the Invention

The present invention addresses the above mentioned problem, and provides an aqueous composition for effectively lightening of hair and especially of artificially colored hair, especially oxidatively colored hair. Further, the present invention provides an aqueous coloring composition for homogenously coloring hair.

The present inventors have unexpectedly found out that an aqueous composition comprising the alkalizing agent as defined above, a guanidine salt and another salt selected from carbonates, bicarbonates, phosphate and hydrogenphosphates with sodium, potassium or ammonium, the concentration of the guanidine salt and the other salt being 3 wt.% or more as defined above, together with an oxidizing agent effectively lightens hair and especially color of artificially colored hair in the presence of an oxidizing agent and provides homogeneous coloration from root to tips with hair dyes especially with oxidative hair dyes.

Thus, a first aspect of the present invention is the aqueous composition for hair as defined above. The aqueous composition of the present invention is preferably free of persalts.

A second aspect of the present invention is a process for treating hair wherein the aqueous composition of the present invention is applied onto hair, left on the hair for 1 to 45 min, and rinsed off from the hair.

A third aspect of the present invention is the use of the aqueous composition of the present invention for lightening hair color and especially of artificially colored hair.

A fourth aspect of the present invention is the kit for treating hair as defined above.

The compositions of the present invention are aqueous compositions and generally comprise 50% by weight or more, calculated to the total composition, water.

The aqueous composition of the present invention comprises at least one alkalizing agent. The suitable ones are ammonia and compounds according to the general formula

R₁R₂R₃N

wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and preferably a mono or polyhydroxyalkyl. Further preferably R₁, R₂ and R₃ are same or different H, C₁ - C₄ alkyl, C₁ - C₄ monohydroxyalkyl, branched C₃ - C₄ monohydroxyalkyl or C₂ - C₄ polyhydroxyalkyl with the condition that at least one of R₁, R₂ and R₃ is not H and preferably a mono or polyhydroxyalkyl.

Examples for such alkalizing agents are ammonia, monoethanolamine, diethanolamine, triethanolamine, monoethanol methylamine, monoethanoldimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures.

Preferred are ammonia, monoethanolamine, diethanolamine, triethanolamine and amino methyl propanol and their mixtures. The even more preferred are ammonia, monoethanolamine and amino methyl propanol and their mixtures.

The concentration of total alkalizing agent in the compositions varies between 1 and 35%, preferably 1 and 30, more preferably 2.5 and 25 and even more preferably 2.5 to 20% by weight calculated to the total the composition.

The aqueous composition of the present invention comprises at least one guanidine salt-which is selected from guanidine carbonate and guanidine phosphate. Particularly preferred is guanidine carbonate.

The aqueous composition of the present invention comprises at least one salt selected from salts of carbonate, bicarbonate, phosphates and hydrogen phosphates with sodium, potassium or ammonium. Suitable salts are sodium carbonate, sodium bicarbonate, trisodium phosphate, disodium hydrogen phosphate, sodium dihydrogen phosphate, potassium carbonate, potassium bicarbonate, tripotassium phosphate, dipotassium hydrogen phosphate, potassium dihydrogen phosphate, ammonium carbonate, ammonium bicarbonate, triammonium phosphate, diammonium hydrogen phosphate and ammonium dihydrogen phosphate. Preferred are sodium bicarbonate, sodium carbonate, potassium bicarbonate, potassium carbonate, ammonium carbonate and ammonium bicarbonate. Particularly preferred are sodium bicarbonate, potassium bicarbonate and ammonium bicarbonate. Ammonium bicarbonate is the especially preferred alkaline salt.

The aqueous composition of the present invention comprises the two salts at a total concentration of 3% or more, preferably 4 to 40%, more preferably 5 to 35% and even more preferably 6 to 30% by weight calculated to the total of the composition.

It has also been found out that the weight ratio between the two salts may play a role in effective lightening of artificially colored hair. The weight ratio between guanidine salt and the second salt is in the range of 10:1 to 1:5, preferably 5:1 to 1:2, more preferably 3.1 to 1:2 and even more preferably 2:1 to 1:1.

The aqueous compositions of the present invention may be in the form of solutions, dispersions, gels and emulsions. Emulsions are a preferred from.

The aqueous emulsion composition preferably comprises one or more fatty alcohol of the general formula

R₄-OH

wherein R₄ is a linear or branched, saturated or unsaturated alkyl chain with 12 to 22 C atoms and at least one emulsifier selected from anionic, non-ionic, cationic and amphoteric surfactants.

Examples for suitable fatty alcohols are myristyl alcohol, cetyl alcohol, stearyl alcohol, oleyl alcohol, octyldodecanol and behenyl alcohol and their mixtures. Preferred is the mixture of cetyl and stearyl alcohol also known as cetearyl alcohol.

The concentration of one or more fatty alcohols is in the range of 1 to 25%, preferably 2.5 to 20%, more preferably 5 to 15% and even more preferably 5 to 12% by weight calculated to total composition prior to mixing with oxidizing agent.

The aqueous emulsion composition of the present invention comprises at least one emulsifier selected from anionic, non-ionic, cationic and amphoteric surfactants. Preferred emulsifying surfactants are anionic, non-ionic and cationic ones and especially preferred are the mixture of anionic and non-ionic surfactants and mixture of cationic and non-ionic surfactants at any ratio. Preferred mixing ratio for the anionic - non-ionic emulsifying surfactant mixture and cationic - non-ionic emulsifying surfactant mixture is in the range of 5:1 to 1:5, more preferably 3:1 to 1:3 and especially 1:1, by weight. It should be noted that incompatibilities can arise when anionic and cationic surfactants are used as the mixture emulsifier which should be taken into account when selecting such a combinations.

In principle, any anionic surfactant is suitable within the meaning of the present invention. Nonlimiting examples are anionic surfactants of the sulfate, sulfonate, carboxylate and alkyl phosphate type, especially, of course, those customarily used as emulsifiers, for example, the known C₁₀-C₁₈-alkyl sulfates, and in particular the respective ethersulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates and their salts.

Further examples for suitable surfactants of the carboxylate type are alkyl polyether carboxylic acids and the salts thereof of the formula

R₅ - (OC₂H₄)ₙ - O - CH₂COOX,

wherein R₅ is a C₈-C₂₀-alkyl group, preferably a C₁₂-C₁₄-alkyl group, n is a number from
1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 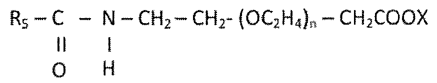 wherein R₅ and X have the above meanings, and n is in particular a number from 1 to 10, preferably 2.5 to 5.

Among the anionic surfactants, alkyl sulfates and/or alkyl ether sulfates are preferable, and among them sodium lauryl or laureth sulfates and their mixtures are more preferred.

Examples for suitable non-ionic surfactants are alkyl polyglucosides of the general formula

R₆ - O - (R₇O)ₙ O - Zₓ

wherein R₆ is an alkyl group with 8 to 18 carbon atoms, R₇ is an ethylene or propylene group, Z is a saccharide group with 5 to 6 carbon atoms, n is a number from 0 to 10 and x is a number between 1 and 5. Examples are decyl polyglucoside and cocoyl polyglucoside, both beeing commercially available.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16".

The average degree of ethoxylation thereby ranges between about 2.5 and about 25, preferably about 10 and about 20.

Among the non-ionic surfactants mentioned above fatty alcohol ethoxylates and fatty acid alkanolamides and their mixtures at any weight ratio are preferable.

As a rule any mono alkyl quaternary ammonium surfactants is suitable for the compositions of the present invention as cationic emulsifying surfactant. With the term mono alkyl it is meant that quaternary ammonium surfactant includes only one alkyl chain which has more than 8 or more C atoms.

Preferably at least one mono alkyl quaternary ammonium surfactant is selected from the compounds with the general formula 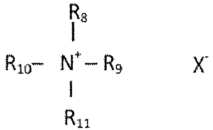 where R₈ is saturated or unsaturated, branched or straight alkyl chain with 8-22 C atoms or

R₁₂ CO NH (CH₂)ₙ

where R₁₂ is saturated or unsaturated, branched or straight alkyl chain with 7-21 C atoms and n has typical value of 1 - 4 or

R₁₂ CO O (CH₂)ₙ

where R₁₂ is saturated or unsaturated, branched or straight alkyl chain with 7-21 C atoms and n has typical value of 1 - 4, and
R₉, R₁₀ and R₁₁ are independent from each other lower alkyl chain with 1 to 4 carbon atoms, hydroxyl alky chain with 1 to 4 C atoms, or ethoxy or propoxy group with number of ethoxy or propoxy groups varying in the range of 1 to 4, and X is chloride, bromide or methosulfate.

Suitable cationic surfactants and or conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride and stearamidopropyltrimethylammonium chloride.

Surfactants are comprised in the aqueous composition at a total concentration of 0.5 to 20%, preferably 1 to 15%, more preferably 2 - 12.5%, and even more preferably 2 to 10% by weight, calculated to the total the composition.

The aqueous composition of the present invention may additionally comprise hair conditioning compounds such as additional oils either of synthetic or of natural ones and cationic polymers. Oils as conditioners according to the present invention are selected from silicone oils either volatile or non-volatile, natural and synthetic oils. Among silicone oils those can be added to the compositions include either volatile or non-volatile dimethicone, dimethiconol, polydimethylsiloxane, any arylated silicones such as phenyl trimethicone, DC fluid ranges from Dow Corning, cyclosiloxanes such as DC 245, as well as aminated siliocnes such as amodimethicone. Synthetic oils include mineral oil such as paraffin oil and petrolatum.

Examples for suitable natural oils are such as argan oil, marula oil, olive oil, almond oil, avocado oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof.

Lipophilic oily compounds such as fatty acid fatty alcohol esters are also suitable for the composition of the present invention. Suitable examples include such according to the general structure

R₁₃ C (O) - O - R₁₄

wherein R₁₃ is an alkyl chain which may be saturated or unsaturated, branched or straight with 7-21 C atoms and R₁₄ is an alkyl chain which may be saturated or unsaturated, branched or straight alkyl chain with 1-22 C atoms or

Examples are such as isopropyl myristate, palmitate, stearate and isostearate, oleyl oleate, isocetyl stearate, hexyl laurate, dibutyl adipate, dioctyl adipate, myristyl myristate, oleyl erucate, cetyl palmitate, etc.

The composition of the present invention can comprise cationic polymers as conditioning agents. Those are cationic cellulose type polymers know as Polymer JR type from Amerchol such as Polyquaternium 10 or cationic guar gum known with trade name Jaguar from Rhone-Poulenc and chemically for example Guar hydroxypropyl trimonium chloride. Furthermore, chitosan and chitin can also be included in the compositions as cationic natural polymers. The cationic polymers mentioned here can also be used.

In this respect, the cationic polymers known with their CTFA category name Polyquaternium have been found to be especially useful. Examples of those include Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46, Polyquaternium 67.

Additionally di alkyl quaternary ammonium surfactants are also suitable conditioners for the aqueous compositions of the present invention. Examples for suitable ones are according to the general structure above wherein R₉ is saturated or unsaturated, branched or straight alkyl chain with 8-22 C atoms or

R₁₂ CO NH (CH₂)ₙ

where R₁₂ is saturated or unsaturated, branched or non-branched alkyl chain with 7-21 C atoms and n has typical value of 1 - 4 or

R₁₂ CO O (CH₂)ₙ

where R₁₂ is saturated or unsaturated, branched or non-branched alkyl chain with 7-21 C atoms and n has typical value of 1 - 4.

The concentration for any of the additional conditioners mentioned above is in the range of 0.01 to 10% by weight, preferably 0.05 - 7.5% by weight, more preferably 0.1 - 5% by weight calculated to the total composition.

The aqueous compositions of the present invention are particularly suitable for homogeneously coloring hair. Suitably, the composition comprises at least one oxidative dye precursor, optionally at least one coupling substance and optionally at least one direct dye.

Examples for suitable oxidative dyestuffs precursors are tetraaminopyrimidines, in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hydroxy-2,4,6-triaminopyrimidine; suitable mono- and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylene-diamine, 2,6-dimethyl-p-phenylenediamine, 2-(2,5-diaminophenyl) ethanol, 1-amino-4-bis-(2'-hydroxyethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-p-hydroxypropyl aminobenzene or the water-soluble salts thereof, pyrazole derivatives such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts. The use of the above mentioned oxidative dye precursors as mixture is also customary in hair coloring area.

The total concentration of the oxidation dyestuff precursors and/or their water soluble salts customarily ranges between about 0.05 % and 5 %, preferably 0.1% and 4 %, in particular 0.1% to 3% by weight, calculated to the total hair dyeing composition prior to mixing with oxidizing agent, whereby these figures are always related to the proportion of free base.

The composition according to the invention optionally comprises at least one coupling substance, which can be selected from resorcinol, 2-methyl resorcinol, 4-chlororesorcinol, 2-amino-4-chlorophenol, 5-amino-4-methoxy-2-methylphenol, 3- amino-phenol, 1-methyl-2-hydroxy-4-aminobenzene, 3-N,N-dimethyl aminophenol, 2.6- dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino-benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

However, this shall not exclude the addition of further developing and coupling substances. In the preferred embodiment of the present invention composition comprise additionally at least one coupling agent.

The weight proportion of the named oxidative dye precursors to the coupling substances ranges between about 1 : 8 to 8 : 1, preferably about 1 : 5 to 5 : 1, in particular 1 : 2 to 2 : 1. In the hair dyeing compositions according to the invention, the coupling substance(s) as reaction partners of the developing substance(s) are present in approximately the same molecular proportions as the developing substances, i.e. in amounts from 0.01 % to 10.0 %, preferably 0.05 % to 7.5 %, in particular 0.1 % to 5 % by weight, calculated to the total composition.

The aqueous composition can optionally comprise direct dyes of neutral, cationic and anionic character. Some examples for suitable cationic dyes are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14 and Basic Yellow 57. According to the invention, suitable cationic dyestuffs are in principal those any available on the market for cosmetic hair colouring applications. For this purpose, special reference is made to the PCT application WO 95/15144 of Ciba-Geigy AG. The content of the PCT application WO 95/15144 is by reference incorporated here.

Examples for suitable direct acting anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Some examples for those suitable neutral dyes (HC dyes), so called nitro dyes, are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4- aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

According to the invention, the composition comprises direct hair dyes at a total concentration of 0.01 to 10%, preferably 0.05 to 7.5%, more preferably 0.1 to 5% by weight calculated to the total the composition.

The compositions of the present invention may further comprise one or more polyol. Suitable ones are glycerine, phytantriol, panthenol, ethyleneglycol, polyethyleneglycols, propylene glycols such as 1,2 propylene glycol, 1,3-propylene glycol and polypropylene glycols.

The concentration of one or more polyol is in the range of 0.1 to 15%, preferably 0.25 to 12.5%, more preferably 0.5 to 10% and even more preferably 1 to 7.5% by weight calculated to the total composition.

The aqueous compositions of the present invention may further comprise one or more thickening agents. The thickening agents may be polymers with thickening properties in aqueous medium, such as nonionic, anionic, cationic and amphoteric polymers. Examples to nonionic ones are those of polysaccharides such as cellulose and its derivatives such as hydroxyethylcellulose, methyl cellulose and ethyl cellulose, guar and its derivatives such as hydroxypropyl guar. Anionic thickening polymers are such as anionic polysaccharides and derivatives thereof, such as alginate, pectin, hyaluronate, anionic gums such as xanthan gum, dehydroxanthan gum, hydroxypropyl xanthan gum, gum arabic, gum karaya or gum tragacanth, or anionic cellulose derivatives such as carboxymethyl cellulose (CMC). Synthetic anionic polymers may also be used as thickeners. Suitable examples are especially the acrylate types such as polyacrylic acid homo or copolymers. Suitable examples for amphoteric polymers are such as copolymers of cationic vinylic or (meth)acrylic monomers with (meth)acrylic acid, such as dimethyldiallylammonium chloride/acrylic acid copolymer (polyquaternium-22, for example, MERQUAT 280, MERQUAT 295; Nalco Company). The polyquaternium compounds listed above as conditioning agents are also suitable as thickeners.

The aqueous composition of the present invention has a pH between 2 and 11, preferably 6 to 11, more preferably 6.8 to 11, even more preferably 8 to 11 and in particular 9 to 10.5.

The aqueous composition of the present invention is mixed with an oxidizing composition comprising at least one oxidizing agent prior to application onto hair. The Examples for suitable oxidizing agents are hydrogen peroxide, urea peroxide, melamin peroxide or perborate salts. The preferred oxidizing agent is hydrogen peroxide, and preferably comprised at a concentration in a range of 1 to 12 % by weight calculated to the total composition.

The pH of the composition comprising at least one oxidizing agent is in the range of 1 to 5, preferably 1.5 to 4 and more preferably 1.5 to 3.

The compositions may additionally comprise an organopolysiloxane wherein at least one silicon atom is linked to an alkylene group having a hetero-atom, in particular a nitrogen atom, with a poly-(N-acyl alkyleneimine) units of the formula 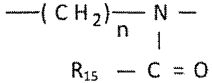 wherein n is a number from 1 to 5 and R₁₅ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 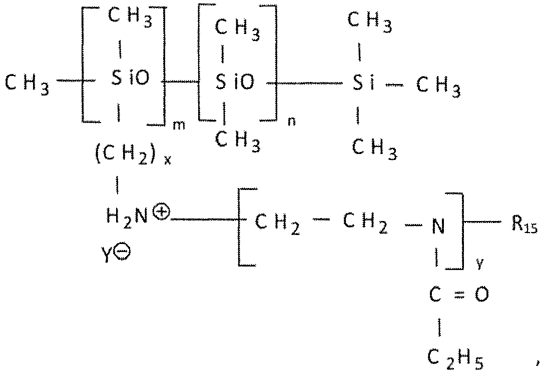 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Another compound that may be comprised in the colouring composition is a ceramide type of compounds according to the general formula 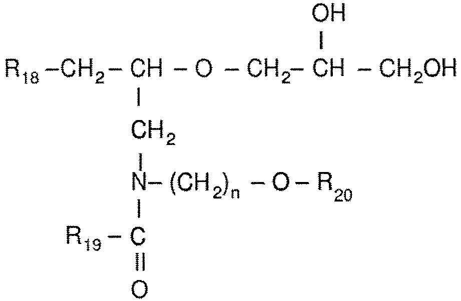 where R₁₈ and R₁₉ are independent from each other alkyl- or alkenyl group with 10 to 22 carbon atoms and R₂₀ is methyl, ethyl, n-propyl or isopropyl group. The concentration of the ceramide type of compound in colouring compositions of the present invention can be in the range of 0.01 to 2 and especially 0.01 to 1% by weight calculated to the total composition.

The compositions according to the present invention can further comprise one or more ubiquinone of the formula. 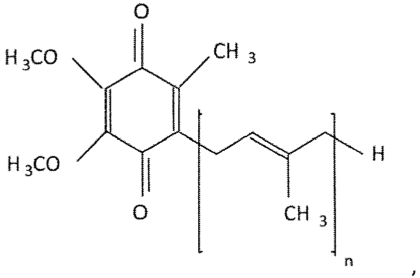 wherein n is a number from 1 to 10. The concentration of ubiquinones in the compositions of the present invention can vary between 0.001% and 10% by weight, calculated to the total composition excluding the oxidizing agent.

The composition of the present invention can certainly comprise compounds for accelerating (catalysts) the oxidative dyeing keratin fibres such as iodine salts i.e. potassium or sodium iodide and/or dihydroxy acetone.

The aqueous compositions of the present invention may further comprise any compounds found regularly in hair cosmetic compositions such as chelating agents, preservatives, reducing agents, fragrance, acids to adjust the pH and other suitable ones.

### Examples

The following examples are to illustrate the invention but not to limit it.

### Example 1

| | Concentration % by weight | | |
|---|---|---|---|
| | 1 | 2 | 3 |
| Ammonium bicarbonate | 15.0 | - | 7.5 |
| Guanidine carbonate | - | 15.0 | 7.5 |
| Ammonia (25%) | 5.6 | 5.6 | 5.6 |
| 1,2-Propylene glycol | 2.5 | 2.5 | 2.5 |
| Water | | to 100 | |

The above compositions had a pH between 10 and 11.

The above compositions were mixed with an aqueous oxidizing composition comprising 12% by weight hydrogen peroxide at a weight ratio of alkaline composition of above to oxidizing composition 2 to 1. The resulting compositions had a pH of 9.2 ± 0.1. The compositions were applied onto hair streaks which were colored with a commercially available hair coloring composition (Topchic 6N) and after processing at ambient temperature for 30 min, rinsed off from hair and hair was dried.

Colors of the hair streaks were measured before and after treating the streaks with above given compositions .From the color intensity (L) values, the differences were obtained between before and after treating the streaks with the above given compositions.

The results were as follows:

| | ΔL |
|---|---|
| Composition 1 | 9.76 |
| Composition 2 | 8.35 |
| Composition 3 | 15.03 |

Form the above results it is beyond any doubt that the two alkaline salts have synergistic effect in lightening color of artificially colored hair.

### Example 2

| | % by weight |
|---|---|
| Cetearyl alcohol | 10.0 |
| Ceteareth-20 | 5.0 |
| Isopropyl palmitate | 1.0 |
| Dimethicone | 1.0 |
| Polyquaternium-10 | 1.0 |
| Cetrimonium chloride | 1.0 |
| Guanidine carbonate | 7.5 |
| Ammonium bicarbonate | 7.5 |
| p-toluenediamine sulphate | 0.1 |
| 2-amino-4-hydroxyethylamino-Anisole | 0.1 |
| Resorcinol | 0.01 |
| 4-chlorresorcinol | 0.01 |
| 1,2 propylene glycol | 3.0 |
| Ammonia (25%) | 8.0 |
| Water | q.s. to 100 |

The above composition was mixed with an aqueous oxidizing composition comprising 12% by weight hydrogen peroxide at a weight ratio of alkaline composition of above to oxidizing composition 2 to 1. The resulting composition had a pH of 9.5 ± 0.05. The composition was applied onto hair streak which was colored with a commercially available hair coloring composition (Colorance 6NN) and after processing at ambient temperature for 30 min rinsed off from hair and hair was dried.

For comparative purposes, two other compositions were produced wherein the one comprises 15% by weight guanidine carbonate (Composition 2-A) and the other one comprised 15% by weight ammonium bicarbonate (Composition 2-B). Hair streaks were colored in the same way as described above.

Color differences (DE) between the non-colored part and the part previously colored was calculated using the well-known equation. The following results were obtained.

| | ΔE |
|---|---|
| Example 2 | 18.74 |
| Composition 2-A | 22.09 |
| Composition 2-B | 22.62 |

From the above results it is beyond any doubt that the hair streak colored with the inventive composition is significantly more homogeneously colored compared to the other two streaks.

### Example 3

| | % by weight |
|---|---|
| Cetearyl alcohol | 10.0 |
| Ceteareth-20 | 5.0 |
| Cetrimonium chloride | 1.0 |
| Guanidine carbonate | 7.5 |
| Ammonium bicarbonate | 7.5 |
| 1,2 propylene glycol | 3.0 |
| p-Toluenediamine sulfate | 1.0 |
| Resorcinol | 0.5 |
| m-amino phenol | 0.01 |
| o-cresol | 0.02 |
| Sodium sulfite | 0.5 |
| Ascorbic acid | 0.2 |
| Fragrance | 0.5 |
| Monoethanolamine | 5.0 |
| Water | q.s. to 100 |

The above composition was mixed with an aqueous oxidizing composition comprising 9% by weight hydrogen peroxide at a weight ratio of above composition to oxidizing composition 2 to 1. The resulting compositions had a pH of 9.5. The composition was applied onto hair streaks and after processing at ambient temperature for 30 min rinsed off from hair and hair was dried. It was observed that hair was homogeneously colored.

### Example 4

| | % by weight |
|---|---|
| Cetearyl alcohol | 10.0 |
| Ceteareth-20 | 4.0 |
| Sodium lauryl sulphate | 4.0 |
| Guanidine carbonate | 7.5 |
| Ammonium bicarbonate | 7.5 |
| 1,2 propylene glycol | 3.0 |
| p-Toluenediamine sulfate | 1.0 |
| Resorcinol | 0.5 |
| Basic red 51 | 0.05 |
| Basic red 76 | 0.05 |
| Basic orange | 0.05 |
| Monoethanolamine | 5.0 |
| Water | q.s. to 100 |

The above composition was mixed with an aqueous oxidizing composition comprising 6% by weight hydrogen peroxide at a weight ratio of above composition to oxidizing composition 1 to 1. The resulting compositions had a pH of 9.5. The composition was applied onto hair streaks and after processing at ambient temperature for 30 min rinsed off from hair and hair was dried. It was observed that hair was homogeneously colored.

### Example 5

| | % by weight |
|---|---|
| Cetearyl alcohol | 10.0 |
| Ceteareth-20 | 4.0 |
| Sodium lauryl sulphate | 4.0 |
| Guanidine carbonate | 7.5 |
| Ammonium bicarbonate | 7.5 |
| 1, 2 propylene glycol | 3.0 |
| p-Toluenediamine sulfate | 1.0 |
| Resorcinol | 0.5 |
| HC Red 3 | 0.1 |
| Basic yellow 87 | 0.05 |
| Monoethanolamine | 5.0 |
| Water | q.s. to 100 |

The above composition was mixed with an aqueous oxidizing composition comprising 9% by weight hydrogen peroxide at a weight ratio of above composition to oxidizing composition 2 to 1. The resulting compositions had a pH of 9.5. The composition was applied onto hair streaks and after processing at ambient temperature for 30 min rinsed off from hair and hair was dried. It was observed that hair was homogeneously colored.

### Example 6

| | % by weight |
|---|---|
| Cetearyl alcohol | 10.0 |
| Ceteareth-20 | 4.0 |
| Sodium lauryl sulphate | 4.0 |
| Guanidine carbonate | 7.5 |
| Ammonium bicarbonate | 7.5 |
| 1,2 propylene glycol | 3.0 |
| p-Toluenediamine sulfate | 1.0 |
| Resorcinol | 0.5 |
| Acid red 92 | 0.1 |
| Basic red 51 | 0.03 |
| Basic yellow 87 | 0.05 |
| HC Yellow 2 | 0.02 |
| Basic brown 16 | 0.01 |
| Amino methyl propanol | 5.0 |
| Water | q.s. to 100 |

The above composition was mixed with an aqueous oxidizing composition comprising 12% by weight hydrogen peroxide at a weight ratio of above composition to oxidizing composition 2 to 1. The resulting compositions had a pH of 9.5. The composition was applied onto hair streaks and after processing at ambient temperature for 30 min rinsed off from hair and hair was dried. It was observed that hair was homogeneously colored.

## Claims

1. An aqueous composition for hair, **characterized in that** it comprises at least one alkalizing agent selected from ammonia and compounds according to the general formula
R₁R₂R₃N
wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and is preferably a mono or polyhydroxyalkyl;
at least one guanidine salt, which is selected from guanidine carbonate or guanidine phosphate;
at least one alkaline salt selected from the salts of carbonate, bicarbonate, phosphate and hydrogenphosphates with sodium, potassium or ammonium; and
at least one oxidizing agent;
wherein the at least one guanidine salt and at least one alkaline salt are comprised in the composition at a total concentration of 3% or more, preferably 5 to 40% by weight, calculated to total composition; the weight ratio of the guanidine salt to the alkaline salt being 10:1 to 1:5; and wherein the composition is preferably free of persalts.

2. The composition according to claim 1 **characterized in that** it comprises 50% by weight or more, calculated to the total composition, water.

3. The composition according to any of the preceding claims **characterized in that** at least one alkalizing agent is selected from ammonia, monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures, preferably the alkalizing agent is ammonia, amino methyl propanol or monoethanolamine or their mixture.

4. The composition according to any of the preceding claims **characterized in that** at least one alkaline salt is selected from sodium carbonate, sodium bicarbonate, trisodium phosphate, disodium hydrogen phosphate, sodium dihydrogen phosphate, potassium carbonate, potassium bicarbonate, tripotassium phosphate, dipotassium hydrogen phosphate, potassium dihydrogen phosphate, ammonium carbonate, ammonium bicarbonate, triammonium phosphate, diammonium hydrogen phosphate and ammonium dihydrogen phosphate, preferably it is ammonium bicarbonate.

5. The composition according to any of the preceding claims **characterized in that** it is an emulsion and preferably comprises one or more of fatty alcohol according to general formula
R₄-OH
wherein R₄ is a linear or branched, saturated or unsaturated alkyl chain with 12 to 22 C atoms and at least one emulsifier selected from anionic, non-ionic, cationic and amphoteric surfactants.

6. The composition according to any of the preceding claims **characterized in that** it comprises conditioning compounds.

7. The composition according to any of the preceding claims **characterized in that** it comprises at least one oxidative dye precursor and optionally at least one coupling substance and optionally at least one direct dye.

8. Process for treating hair wherein an aqueous composition according to claims 1 to 7 is applied onto hair and after leaving on the hair for 1 to 45 min, rinsed off from hair.

9. Use of an aqueous composition according to claims 1 to 7 for treating hair.

10. Kit for hair comprising two or more aqueous compositions wherein one of the compositions is an aqueous composition comprising at least one alkalizing agent selected from ammonia and compounds according to the general formula
R₁R₂R₃N
wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and is preferably a mono or polyhydroxyalkyl;
at least one guanidine salt, which is selected from guanidine carbonate or guanidine phosphate;
at least one alkaline salt selected from the salts of carbonate, bicarbonate, phosphate and hydrogenphosphates with sodium, potassium or ammonium; wherein at least one guanidine salt and at least one alkaline salt are comprised in the composition at a total concentration of 3% or more, preferably 5 to 40% by weight, calculated to total composition, the weight ratio of the guanidine salt to the alkaline salt being 10:1 to 1:5; and
wherein the composition is preferably free of persalts; and
the other composition is an aqueous composition comprising at least one oxidizing agent.

## Patentansprüche

1. Wässrige Zusammensetzung für Haare, **dadurch gekennzeichnet, dass** sie mindestens ein Alkalisierungsmittel, ausgewählt aus Ammoniak und Verbindungen gemäß der allgemeinen Formel
R₁R₂R₃N
worin R₁, R₂ und R₃, die gleich oder verschieden sind, H, C₁-C₆-Alkyl, C₁-C₆-Monohydroxyalkyl, verzweigtes C₃-C₆-Monohydroxyalkyl oder C₂-C₆-Polyhydroxyalkyl sind, mit der Bedingung, dass mindestens eines von R₁, R₂ und R₃ nicht H ist und bevorzugt ein Mono- oder Polyhydroxyalkyl ist;
mindestens ein Guanidin-Salz, ausgewählt aus Guanidin-Carbonat oder Guanidin-Phosphat;
mindestens ein alkalisches Salz, ausgewählt aus Carbonat-, Bicarbonat-, Phosphat- und Hydrogenphosphat-Salzen mit Natrium, Kalium oder Ammonium; und
mindestens ein Oxidationsmittel umfasst;
wobei das mindestens eine Guanidin-Salz und das mindestens eine alkalische Salz in der Zusammensetzung in einer Gesamtkonzentration von 3 Gew.-% oder mehr, bevorzugt 5 bis 40 Gew.-%, berechnet auf die Gesamtzusammensetzung, enthalten sind; das Gewichtsverhältnis des Guanidin-Salzes zu dem alkalischen Salz 10:1 bis 1:5 ist; und wobei die Zusammensetzung bevorzugt frei von Persalzen ist.

2. Zusammensetzung gemäß Anspruch 1, **dadurch gekennzeichnet, dass** sie 50 Gew.-% oder mehr, berechnet auf die Gesamtzusammensetzung, Wasser umfasst.

3. Zusammensetzung gemäß einem der vorhergehenden Ansprüche, **dadurch gekennzeichnet, dass** mindestens ein Alkalisierungsmittel aus Ammoniak, Monoethanolamin, Diethanolamin, Triethanolamin, Monoethanolmethylamin, Monoethanoldimethylamin, Diethanolmethylamin, Monoethanolethylamin, Monoethanoldiethylamin, Diethanolethylamin, Monoethanolpropylamin, Monoethanoldipropylamin, Diethanolpropylamin, Monoethanolbutylamin, Diethanolbutylamin und Aminomethylpropanol und Mischungen davon ausgewählt ist, bevorzugt ist das Alkalisierungsmittel Ammoniak, Aminomethylpropanol oder Monoethanolamin oder Mischungen davon.

4. Zusammensetzung gemäß einem der vorhergehenden Ansprüche, **dadurch gekennzeichnet, dass** mindestens ein alkalisches Salz aus Natriumcarbonat, Natriumbicarbonat, Trinatriumphosphat, Dinatriumhydrogenphosphat, Natriumdihydrogenphosphat, Kaliumcarbonat, Kaliumbicarbonat, Trikaliumphosphat, Dikaliumhydrogenphosphat, Kaliumdihydrogenphosphat, Ammoniumcarbonat, Ammoniumbicarbonat, Triammoniumphosphat, Diammoniumhydrogenphosphat und Ammoniumdihydrogenphosphat ausgewählt ist, bevorzugt ist es Ammoniumbicarbonat.

5. Zusammensetzung gemäß einem der vorhergehenden Ansprüche, **dadurch gekennzeichnet, dass** sie eine Emulsion ist und bevorzugt einen oder mehrere Fettalkohole gemäß der allgemeinen Formel
R₄-OH
worin R₄ eine lineare oder verzweigte, gesättigte oder ungesättigte Alkylkette mit 12 bis 22 C-Atomen ist, und mindestens einen Emulgator, ausgewählt aus anionischen, nichtionischen, kationischen und amphoteren Tensiden, umfasst.

6. Zusammensetzung gemäß einem der vorhergehenden Ansprüche, **dadurch gekennzeichnet, dass** sie konditionierende Verbindungen umfasst.

7. Zusammensetzung gemäß einem der vorhergehenden Ansprüche, **dadurch gekennzeichnet, dass** sie mindestens einen oxidativen Farbstoffvorläufer und optional mindestens eine Kupplungssubstanz und optional mindestens einen Direktfarbstoff umfasst.

8. Verfahren zur Behandlung von Haaren, bei dem eine wässrige Zusammensetzung gemäß den Ansprüchen 1 bis 7 auf die Haare aufgebracht wird und nach dem Belassen auf den Haaren für 1 bis 45 min von den Haaren abgespült wird.

9. Verwendung einer wässrigen Zusammensetzung gemäß den Ansprüchen 1 bis 7 zur Behandlung von Haaren.

10. Haar-Kit, umfassend zwei oder mehr wässrige Zusammensetzungen, wobei eine der Zusammensetzungen eine wässrige Zusammensetzung ist, die mindestens ein Alkalisierungsmittel, ausgewählt aus Ammoniak und Verbindungen gemäß der allgemeinen Formel
R₁R₂R₃N
worin R₁, R₂ und R₃, die gleich oder verschieden sind, H, C₁-C₆-Alkyl, C₁-C₆-Monohydroxyalkyl, verzweigtes C₃-C₆-Monohydroxyalkyl oder C₂-C₆-Polyhydroxyalkyl sind, mit der Bedingung, dass mindestens eines von R₁, R₂ und R₃ nicht H ist und bevorzugt ein Mono- oder Polyhydroxyalkyl ist;
mindestens ein Guanidin-Salz, ausgewählt aus Guanidin-Carbonat oder Guanidin-Phosphat;
mindestens ein alkalisches Salz, ausgewählt aus Carbonat-, Bicarbonat-, Phosphat- und Hydrogenphosphat-Salzen mit Natrium, Kalium oder Ammonium, umfasst;
wobei das mindestens eine Guanidin-Salz und das mindestens eine alkalische Salz in der Zusammensetzung in einer Gesamtkonzentration von 3 Gew.-% oder mehr, bevorzugt 5 bis 40 Gew.-%, berechnet auf die Gesamtzusammensetzung, enthalten sind; das Gewichtsverhältnis des Guanidin-Salzes zu dem alkalischen Salz 10:1 bis 1:5 ist; und
wobei die Zusammensetzung bevorzugt frei von Persalzen ist; und
die andere Zusammensetzung eine wässrige Zusammensetzung ist, die mindestens ein Oxidationsmittel umfasst.

## Revendications

1. Composition aqueuse pour les cheveux **caractérisée en ce qu'**elle comprend au moins un agent alcalinisant choisi parmi l'ammoniac et les composés en accordance avec la formule générale
R₁R₂R₃N
dans laquelle R₁, R₂ et R₃ sont identiques ou différents H, alkyle en C₁-C₆, monohydroxyalkyle en C₁-C₆, polyhydroxylakyle en C₂-C₆ ou monohydroxyalkyle en C₃-C₆ ramifié à la condition qu'au moins l'un de R₁, R₂ et R₃ ne soit pas H et soit préférablement un mono ou polyhydroxyalkyle ;
au moins un sel de guanidine, qui est choisi parmi le carbonate de guanidine et le phosphate de guanidine ;
au moins un sel alcalin choisi parmi les sels de carbonate, bicarbonate, phosphate et hydrogène phosphates avec du sodium, du potassium ou de l'ammonium ; et
au moins un agent oxydant ;
dans laquelle le au moins un sel de guanidine et le au moins un sel alcalin sont compris dans la composition à une concentration totale de 3% ou plus, préférablement de 5 à 40% en poids, calculés sur la base de la composition totale; le rapport pondéral du au moins un sel de guanidine à l'au moins un sel alcalin étant de 10:1 à 1:5 ; et dans laquelle la composition est préférablement sans persels.

2. La composition selon la revendication 1 **caractérisée en ce qu'**elle comprend 50% en poids ou plus, calculés par rapport à la composition totale, d'eau.

3. La composition selon l'une quelconque des revendications précédentes **caractérisée en ce qu'**au moins un agent alcalinisant choisi parmi l'ammoniac, la monoéthanolamine, la diéthanolamine, la triéthanolamine, la méthylamine de monoéthanol, la monoéthanoldiméthylamine, la di-éthanolméthylamine, la monoéthanoléthylamine, la monoéthanoldiéthylamine, la diéthanoléthylamine, la monoéthanolpropylamine, la monoéthanoldipropylamine, la diéthanolpropylamine, la monoéthanolbutylamine, la diéthanolbutylamine et l'amino méthyl propanol et leurs mélanges, préférablement l'agent alcalinisant est l'ammoniac, l'amino méthyl propanol ou la monoéthanolamine et leurs mélanges.

4. La composition selon l'une quelconque des revendications précédentes **caractérisée en ce qu'**au moins un sel alcalin est choisi parmi le carbonate de sodium, le bicarbonate de sodium, le phosphate de trisodium, le phosphate d'hydrogène de disodium, le phosphate de dihydrogène de sodium, le carbonate de potassium, le bicarbonate de potassium, le phosphate de tripotassium, le phosphate d'hydrogène de dipotassium, le phosphate de dihydrogène de potassium, le carbonate d'ammonium, le bicarbonate d'ammonium, le phosphate de triammonium, le phosphate d'hydrogène de diammonium et le phosphate de dihydrogène d'ammonium, préférablement c'est du bicarbonate d'ammonium.

5. La composition selon l'une quelconque des revendications précédentes **caractérisée en ce qu'**elle est une émulsion et préférablement comprend un ou plusieurs alcool(s) gras en accordance avec la formule générale
R₄-OH
dans laquelle R₄ est une chaîne alkyle insaturée ou saturée avec 12 à 22 atomes de carbone, linéaire ou ramifiée, et au moins un émulsifiant choisi parmi les tensioactifs amphotères, cationiques, non-ioniques et anioniques.

6. La composition selon l'une quelconque des revendications précédentes **caractérisée en ce qu'**elle comprend des composés de conditionnement.

7. La composition selon l'une quelconque des revendications précédentes **caractérisée en ce qu'**elle comprend au moins un précurseur de colorant oxydatif et optionnellement au moins une substance de couplage et optionnellement au moins un colorant direct.

8. Procédé pour le traitement des cheveux dans lequel une composition aqueuse selon les revendications 1 à 7 est appliquée sur les cheveux et après avoir été laissée sur les cheveux pendant 1 à 45 minutes, est rincée des cheveux.

9. Utilisation d'une composition aqueuse selon les revendications 1 à 7 pour le traitement des cheveux.

10. Kit pour les cheveux comprenant deux ou plusieurs compositions aqueuses dans lequel l'une des compositions est une composition aqueuse comprenant :
au moins un agent alcalinisant choisi parmi l'ammoniac et les composés en accordance avec la formule générale
R₁R₂R₃N
dans laquelle R₁, R₂ et R₃, qui peuvent être identiques ou différents, sont choisis parmi H, alkyle en C₁-C₆, monohydroxyalkyle en C₁-C₆, polyhydroxylakyle en C₂-C₆ ou monohydroxyalkyle en C₃-C₆ ramifié à la condition qu'au moins l'un de R₁, R₂ et R₃ ne soit pas H et soit préférablement un mono ou polyhydroxyalkyle ;
au moins un sel de guanidine, qui est choisi parmi le carbonate de guanidine et le phosphate de guanidine ;
au moins un sel alcalin choisi parmi les sels de carbonate, bicarbonate, phosphate et hydrogène phosphates avec du sodium, du potassium ou de l'ammonium ; et
dans laquelle le au moins un sel de guanidine et le au moins un sel alcalin sont compris dans la composition à une concentration totale de 3% ou plus, préférablement de 5 à 40% en poids, calculés sur la base de la composition totale; le rapport pondéral du au moins un sel de guanidine à l'au moins un sel alcalin étant de 10:1 à 1:5 ; et
dans laquelle la composition est préférablement sans persels ; et
l'autre composition est une composition aqueuse comprenant un agent oxydant.
